# FASCICULE DE BREVET EUROPEEN

(11) **EP 3 946 041 B1**
(45) Date de publication et mention de la délivrance du brevet: **04.11.2026**
(21) Numéro de dépôt: 20716472.4
(22) Date de dépôt: 03.04.2020
(51) Int. Cl.: A61B 5/053, A61B 5/00, A61B 5/01

(54) **DISPOSITIF MEDICAL POUR LA DETECTION D'UNE TUMEUR MAMMAIRE, PROCÉDÉ, PROGRAMME D'ORDINATEUR ET SUPPORT D'ENREGISTREMENT ASSOCIÉS**
MEDIZINISCHE VORRICHTUNG ZUM NACHWEIS EINES BRUSTTUMORS
MEDICAL DEVICE FOR DETECTING A MAMMARY TUMOR

(30) Priorité: 05.04.2019 FR 1903655
(43) Date de publication de la demande: 09.02.2022
(73) Titulaire: ONCELIA, 42000 Saint-Étienne (FR)
(72) Inventeur: MORENO, Marie-Valérie, 69250 Neuville-Sur-Saône (FR)
(74) Mandataire: Ipsilon
(86) Numéro de dépôt international: PCT/EP2020/059646
(87) Numéro de publication internationale: WO 2020/201546

(56) Documents cités:
- US-A1- 2013 184 606
- US-A1- 2018 160 952
- SUNJOO HONG ET AL: "A 4.9 m[Omega]-Sensitivity Mobile Electrical Impedance Tomography IC for Early Breast-Cancer Detection System", IEEE JOURNAL OF SOLID-STATE CIRCUITS, vol. 50, no. 1, 1 January 2015 (2015-01-01), USA, pages 245 - 257, XP055709705, ISSN: 0018-9200, DOI: 10.1109/JSSC.2014.2355835
- ALI ZARAFSHANI: "Feasibility Test of Quantitative Assessment of Breast Density Based on Dielectric Impedance Spectroscopy", JOURNAL OF APPLIED BIOTECHNOLOGY & BIOENGINEERING, vol. 2, no. 6, 4 April 2017 (2017-04-04), XP055663029, DOI: 10.15406/jabb.2017.02.00047
- TERESA MALECKA-MASSALSKA ET AL: "Bioimpedance vector pattern in women with breast cancer detected by bioelectric impedance vector analysis. Preliminary observations", ANNALS OF AGRICULTURAL AND ENVIRONMENTAL MEDICINE : AAEM, 30 November 2012 (2012-11-30), Poland, pages 697 - 700, XP055474621, Retrieved from the Internet <URL:https://epo.summon.serialssolutions.com/2.0.0/link/0/eLvHCXMwtV1LaxsxEBZNCiWXkDaheRV0aE5lg3al9cpHxyTuxZeyl56CXhu2edjYTsC-5o9nRtqHSCg0h14WW2t00HyM5_s0D0K-A-kZ5lbZxA4KAQRFV4mqnIZALrWSW66Z71vweyzKSfFrgtMa2tFd_dp_tTSsga2xcvYd1u42hQX4DDaHJ1gdnv9k94t6VkMobH0pwJPX5LF5Kup-qG34jgtBfdWYj77CtC_jx4TjdUKIR3U9C-N> [retrieved on 20180514]

## Description

L'invention concerne un dispositif médical suivant la revendication 1.

Le cancer du sein est le cancer le plus fréquemment observé chez les femmes en Europe et aux Etats-Unis. Cette maladie reste la première cause de décès par cancer chez les femmes en 2012. Or, s'il est dépisté à un stade précoce, ce cancer peut être guéri dans 9 cas sur 10.

Le dépistage du cancer du sein peut être réalisé par la mammographie. En France, cet examen est préconisé et gratuit tous les deux ans après 50 ans. La mammographie est cependant accusée d'entraîner des sur-diagnostics, c'est- à-dire une détection de lésions précancéreuses qui n'auraient jamais évolué.

De plus, l'espace entre deux mammographies est important et il est possible qu'une lésion précancéreuse ait le temps de grossir suffisamment pendant cet intervalle pour représenter un danger.

On connaît également certaines techniques utilisant les rayons X afin de réaliser de l'imagerie du sein pour la détection de tumeur. Cependant, ces rayons comprennent un effet cancérogène, surtout pour les femmes en dessous de 50 ans dont les seins sont plus denses, nécessitant une plus forte dose de radiation.

On connait aussi des solutions dans le document "Feasibility Test of Quantitative Assessment of Breast Density Based on Dielectric Impedance Spectroscopy" de ALI ZARAFSHANI publié au JOURNAL OF APPLIED BIOTECHNOLOGY &

BIOENGINEERING, vol. 2, no. 6, 4 avril 2017, le document "A 4.9 m[Omega]-Sensitivity Mobile Electrical Impedance Tomography IC for Early Breast-Cancer Detection System", de Sunjoo Hong ET AL, publié dans le IEEE Journal of Solid-State Circuits, vol. 50, no. 1, du1 janvier 2015, ou encore les documents US 2013/184606 A1, US 2018/160952 A1.

De nombreux chercheurs se sont intéressés à la détection des cancers du sein par bioimpédance consistant à mesurer l'impédance dans le volume des

tissus du sein afin de réaliser de l'imagerie. Toutefois, cette technique reste une solution ponctuelle et coûteuse.

Le but de l'invention est de fournir un dispositif médical pour l'estimation d'un risque de présence d'une tumeur cancéreuse remédiant aux inconvénients ci-dessus et améliorant les dispositifs connus de l'art antérieur. Plus précisément, l'invention a pour objet de réaliser un dispositif médical qui soit simple et fiable et qui permettent une estimation d'un risque de présence de tumeur de manière fréquente et sans la présence d'un personnel médical.

L'invention concerne un dispositif médical selon l'objet de la revendication 1.

Dans un mode de réalisation, le jeu d'électrodes comprend au moins une première électrode au sommet du au moins un bonnet et au moins une deuxième électrode au niveau de la base du au moins un bonnet.

Dans un mode de réalisation, la au moins une première électrode comprend une première électrode de mesure et une première électrode d'injection et/ou en ce que la au moins une deuxième électrode comprend une deuxième électrode d'injection et une deuxième électrode de mesure.

Dans un mode de réalisation, chaque électrode d'injection et chaque électrode de mesure s'étend au moins partiellement autour du bonnet, préférentiellement s'étend sur au moins 40%, voire sur au moins 80%, de la périphérie du bonnet.

Le bonnet peut comprendre au moins une électrode à proximité de la base du bonnet de forme totalement ou partiellement annulaire, et au moins une électrode à proximité du sommet du bonnet, et au moins une branche flexible ou semi-élastique reliant lesdites électrodes, et le bonnet présente une structure flexible de sorte à permettre le maintien desdites électrodes au contact d'un sein et le maintien du bonnet au contact d'un sein.

Dans un mode de réalisation, le dispositif médical comprend en outre une source électrique pour appliquer une tension entre la première électrode et la deuxième électrode et comprend un élément de mesure de tension pour mesurer la tension entre la première et la deuxième électrode.

Dans un mode de réalisation, les deux électrodes de mesure sont agencées entre les deux électrodes d'injection.

Dans un mode de réalisation, le bonnet comprend en outre une pluralité d'électrodes ponctuelles, préférentiellement agencées en réseau, pour réaliser une cartographie d'impédance dans le volume du sein.

Dans un mode de réalisation, le dispositif médical comprend des éléments matériels et/ou logiciels mettant en oeuvre un procédé de calcul d'un score de risque. Ledit procédé de calcul d'un score de risque comprend les étapes suivantes :
- une mesure d'une impédance et/ou d'une résistance entre le sommet du sein et la base du sein ;
- une estimation de la densité mammaire à partir de cette mesure ;
- une comparaison de ladite estimation à une valeur de référence ;
- un calcul d'un score de risque en fonction de cette comparaison.

Dans un mode de réalisation, ledit procédé de calcul d'un score comprend les étapes suivantes :
- une mesure d'une impédance entre le sommet du sein et la base du sein ;
- une comparaison de ladite mesure à une valeur de référence ;
- un calcul d'un score de risque en fonction de cette comparaison.

Dans un mode de réalisation, ledit procédé de calcul d'un score comprend les étapes suivantes :
- une mesure d'une première température de l'utilisatrice au niveau du sein;
- une comparaison de la première température à une température de référence.

Le score de risque est alors calculé en fonction de la comparaison de température.

Dans un mode de réalisation, ledit procédé de calcul d'un score comprend une mesure de la température corporelle de l'utilisatrice et le score de risque est calculé en fonction de la mesure de la température corporelle de l'utilisatrice.

Dans un mode de réalisation, la valeur de référence et/ou la température de référence est déterminée à partir d'au moins une mesure précédente ou par une mesure sur le deuxième sein ou sur une autre partie du corps.

Dans un mode de réalisation, le dispositif médical comprend des éléments matériels et/ou logiciels mettant en oeuvre un procédé de réalisation d'une cartographie du volume du sein par bioimpédance.

L'invention concerne également un produit programme d'ordinateur comprenant des instructions de code de programme enregistrées sur un support lisible par ordinateur pour mettre en oeuvre les étapes d'un procédé de calcul d'un score de risque tel que décrit ci-avant.

L'invention concerne aussi un support d'enregistrement des données lisible par un ordinateur, sur lequel est enregistré un programme d'ordinateur selon l'invention.

L'invention concerne enfin un procédé d'estimation de la densité mammaire d'un sein comprenant les étapes suivantes :
- le placement sur ledit sein d'un bonnet d'un dispositif médical selon l'invention ;
- la mesure d'une impédance entre le sommet du sein et la base du sein ;
- l'estimation de la densité mammaire à partir de cette mesure d'impédance.

L'invention est plus précisément définie par les revendications.

Ces objets, caractéristiques et avantages de la présente invention seront exposés en détail dans la description suivante de modes d'exécution particuliers faits à titre non-limitatif en relation avec les figures jointes parmi lesquelles :
La figure 1 représente un bonnet d'un dispositif médical selon un mode de réalisation de l'invention comprenant 4 électrodes, deux au sommet et deux à la base du bonnet pour réaliser des mesures d'impédances pour estimer la densité mammaire.
La figure 2 représente un bonnet d'un dispositif médical selon un mode de réalisation dans lequel le bonnet comprend un capteur de température.
La figure 3 représente un bonnet d'un dispositif médical selon un mode de réalisation dans lequel le bonnet comprend un maillage d'électrodes sur la surface du bonnet pour réaliser une cartographie par tomographie d'impédance du sein.
La figure 4 représente un soutien-gorge d'un dispositif médical selon un mode de réalisation de l'invention comprenant deux bonnets munis d'électrodes pour estimer la densité mammaire de chaque sein.
La figure 5 représente un soutien-gorge d'un dispositif médical selon un mode de réalisation de l'invention comprenant deux bonnets munis d'un réseau d'électrodes ponctuelles pour réaliser une cartographie par tomographie d'impédance du sein.
La figure 6 est un schéma des moyens électroniques du dispositif médical selon un mode de réalisation de l'invention.
La figure 7 est un schéma illustrant la méthodologie de tomographie par impédance électrique.
La figure 8 est un schéma illustrant la méthodologie de mesure d'impédance tripolaire dans le volume du sein, ce schéma illustre une vue de coupe du bonnet de profil.
La figure 9 représente un bonnet d'un dispositif médical selon un mode de réalisation dans lequel le bonnet comprend une pluralité de capteurs de température sur la surface du bonnet.
La figure 10 représente un schéma des variations des mesures de résistance et de réactance du sein, induites par la proportion de tissu fibro-glandulaires et conjonctifs par rapport aux tissus adipeux, et/ou la présence de tumeurs.
La figure 11 représente un bonnet d'un dispositif médical selon un mode de réalisation.
La figure 12 représente un dispositif médical selon un mode de réalisation.

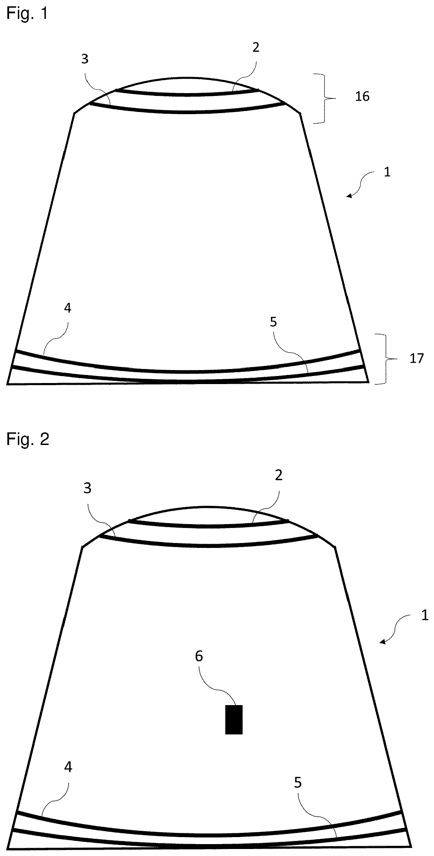

Un exemple d'un dispositif médical selon un mode de réalisation de l'invention est décrit ci-après en référence aux figures 1 à 12.

Le dispositif médical 10 comprend un ou deux bonnets 1 destinés à couvrir un sein tel qu'un bonnet de soutien-gorge ou d'une brassière. Chaque bonnet 1 peut être un bonnet de soutien-gorge 18, conçu pour soutenir et optionnellement protéger le sein. Autrement dit, le dispositif médical peut comprendre un bonnet conventionnel de soutien-gorge, comprenant une partie en textile prévue pour le maintien d'un sein et des éléments particuliers remplissant la fonction médicale, qui seront décrits par la suite, intégrés dans le textile du bonnet ou simplement juxtaposés au textile. En variante, le bonnet 1 peut être formé par les seuls éléments remplissant la fonction médicale, par exemple en l'absence de tout textile. Dans tous les cas, le bonnet est un tel qu'il couvre au moins partiellement un sein, de sa base à son sommet, en faisant presque le tour de la base du sein. Il peut comprendre des zones ajourées, c'est-à-dire qu'il ne couvre pas nécessairement toute la surface d'un sein entre sa base et son sommet. Avantageusement, le bonnet 1 est dimensionné pour couvrir un sein suffisamment pour se maintenir seul au contact du sein.

Le dispositif médical 10 peut donc comprendre un soutien-gorge 18 traditionnel comprenant un ou deux bonnets 1 . Lorsque le soutien-gorge 18 (ou plus simplement le dispositif médical 10) comprend un deuxième bonnet 1, celui-ci est similaire au premier bonnet 1 . Pour faciliter la compréhension de la description, on décrira uniquement le premier bonnet 1

Le bonnet comprend notamment des moyens de mesure de l'impédance et/ou de résistance des tissus mammaires dans le volume du sein. En remarque, nous entendons par mesures d'impédance et/ou de résistance dans tout le document toute mesure directe et indirecte permettant d'obtenir directement ces grandeurs, comme des mesures de tension, de variations de tension en fonction du temps, de courant et/ou de variations de courant en fonction du temps.

Le dispositif médical 10 permet une estimation de la densité mammaire. Le sein comprend un mélange de tissus mous (aussi appelés tissus fibro-glandulaires et conjonctifs dans la suite de la description) et de tissus adipeux (aussi appelés tissus adipeux dans la suite de description), ayant une conductivité et une capacité différentes.

Le dispositif médical 10 permet également la détection d'un risque de présence d'une tumeur cancéreuse dans le sein. En effet, les tissus cancéreux ont une conductivité et une capacité supérieures à celle des tissus sains.

L'invention permet ainsi de réaliser des mesures de résistance, de réactances et/ou d'impédance dans le volume du sein pour déterminer la densité mammaire et/ou détecter un risque de présence d'une tumeur cancéreuse.

Dans un mode de réalisation, le dispositif médical 10 permet une mesure de la température par zone cutanée sur un sein et/ou sur chaque sein et une mesure de la température corporelle.

Selon un mode de réalisation, le soutien-gorge 18 est conçu pour être porté par une utilisatrice (ou toute personne ou animal dont on voudrait prévenir un cancer du sein) de la même manière qu'un soutien-gorge classique. Le soutien-gorge 18 est conçu pour être porté régulièrement par l'utilisatrice (tous les jours ou au moins plusieurs fois par mois) de manière à ce que le dispositif médical 10 puisse effectuer des mesures régulièrement.

Le bonnet 1 est destiné à recouvrir au moins partiellement un sein, préférentiellement à recouvrir l'intégralité du sein. Le bonnet 1 est conçu pour recouvrir au moins 80%, voire 90% de la surface du sein. En variante, il peut être ajouré, et couvrir une surface inférieure. Le bonnet 1 est conçu pour recouvrir le sein au niveau de sa base et au niveau de son sommet. Le bonnet 1 peut comprendre un textile élastique de manière à ce que ledit textile soit en contact avec la peau du sein

L'estimation de la densité mammaire peut être réalisée par quantification de la masse graisseuse par rapport à la masse de tissus mous par impédance.

Le bonnet 1 comprend une ou deux électrodes 2, 3 agencées au sommet 16 du bonnet 1 et une ou deux électrodes 4, 5 agencées à la base 17 du bonnet 1 de manière à permettre de réaliser des mesures d'impédance et/ou des mesures de résistance dans le volume du sein, entre sa base et son sommet. Les électrodes 2, 3, 4, 5, peuvent comprendre des électrodes sèches et/ou des électrodes Gel. Les électrodes peuvent également comprendre des électrodes textiles.

Cette mesure d'impédance et/ou de résistance permet avantageusement de déterminer au moins une estimation de la densité mammaire. Cette mesure d'impédance peut permettre également de déterminer une estimation du risque de présence d'une tumeur cancéreuse dans le volume du sein, notamment à partir de l'estimation de la densité mammaire.

Les électrodes sont agencées de manière à réaliser des mesures d'impédance dans le volume du sein. Les électrodes sont agencées sur le bonnet de manière à être en contact avec la peau du sein de l'utilisatrice.

Dans un mode de réalisation préférentiel illustré sur la figure 1 , le premier jeu d'électrodes comprend quatre électrodes.

Le premier jeu d'électrodes comprend deux électrodes d'injection 2, 5 pour injecter un courant dans le volume du sein et deux électrodes de mesure 3, 4 pour mesurer une tension ou une impédance dans le volume du sein. Lorsqu'un courant alternatif est injecté entre les deux électrodes d'injection 2, 5, le courant parcourt le volume entre ces deux électrodes selon des lignes formant une forme ovoïdale.

Les électrodes de mesure 3, 4 permettent de mesurer le potentiel résultant entre ces deux électrodes de mesure 3, 4 dans le volume du sein. L'application de la loi d'Ohm permet dés lors de déterminer l'impédance bioélectrique et/ou la résistance entre les électrodes de mesure à partir du courant appliqué connu et de la tension mesurée.

Afin de réaliser ces mesures sur le plus grand volume possible du sein, les électrodes sont disposées au niveau du sommet 16 (plus généralement à proximité du sommet du bonnet 1 ) et sur la base 17 du bonnet 1 (plus généralement à proximité de la base du bonnet 1 ). Préférentiellement, chaque électrode 2, 3, 4, 5 s'étend longitudinalement autour du bonnet 1 de manière à former au moins partiellement un cercle d'électrodes autour du sein. Dans un mode de réalisation, chacune des électrodes de mesure et d'injection s'étend autour du bonnet sur au moins 40%, voire sur au moins 60%, 70%, 80% ou 90%, de la périphérie du bonnet, préférentiellement en forme de cercle ou d'arc de cercle. Dans un mode de réalisation, chaque électrode 2, 3, 4, 5 peut comprendre une pluralité d'électrodes proches les unes les autres et formant au moins partiellement un cercle s'étendant autour du bonnet sur au moins 50%, voire sur au moins 80% ou 90%, de la périphérie du bonnet. Finalement, chaque électrode présente une forme annulaire, éventuellement tronquée.

Cette forme circulaire ou d'arc de cercle autour de la périphérie du bonnet 1 permet avantageusement d'injecter un courant et de réaliser des mesures dans la totalité du volume du sein.

Plus précisément, le premier jeu d'électrode comprend une première électrode de mesure 3 et une première électrode d'injection 2 au niveau du sommet 16 du bonnet 1 et une deuxième électrode d'injection 5 et une deuxième électrode de mesure 4 au niveau de la base 17 du bonnet.

Dans un mode de réalisation, les électrodes situées au sommet du bonnet sont des électrodes ponctuelles disposées sur le sommet du bonnet. Ainsi, le volume dans lequel sont réalisées les mesures bioélectriques est augmenté.

Par « le sommet du bonnet », on entend une première zone 16 faisant le tour du bonnet à l'extrémité distale du bonnet, destinée à être sur le mamelon du sein.

Par « la base du bonnet », on entend ici une deuxième zone 17 faisant le tour du bonnet à proximité du thorax, à l'extrémité proximale du bonnet.

Les deux premières électrodes 2, 3 sont destinées à être en contact avec le sein au niveau du mamelon ou du sommet du sein. Les deux deuxièmes électrodes 4, 5 sont destinées à être en contact avec le sein au niveau de sa base.

La disposition des électrodes de mesure 3, 4 et d'injection 2, 5 permet de réaliser des mesures sur la plus grande partie du volume du sein possible. Les deux premières électrodes 2, 3 sont préférentiellement disposées à une distance inférieure à 5 cm, préférentiellement inférieure à 3 cm du sommet 16 du bonnet 1, c'est-à-dire à proximité du sommet du bonnet. Les deux deuxièmes électrodes 3, 4 sont préférentiellement disposées à une distance inférieure à 2 cm, préférentiellement inférieure à 1 cm de la base 17 du bonnet 1 , c'est-à-dire à proximité de la base du bonnet. Les deux premières électrodes 2, 3 sont préférentiellement disposées à une distance inférieure à 3 cm, préférentiellement inférieure à 2 cm l'une de l'autre. Les deux deuxièmes électrodes 4, 5 sont préférentiellement disposées à une distance inférieure à 3 cm, préférentiellement inférieure à 2 cm l'une de l'autre. Les électrodes de mesures 3, 4 sont agencées au moins entre les deux électrodes d'injection 2, 5.

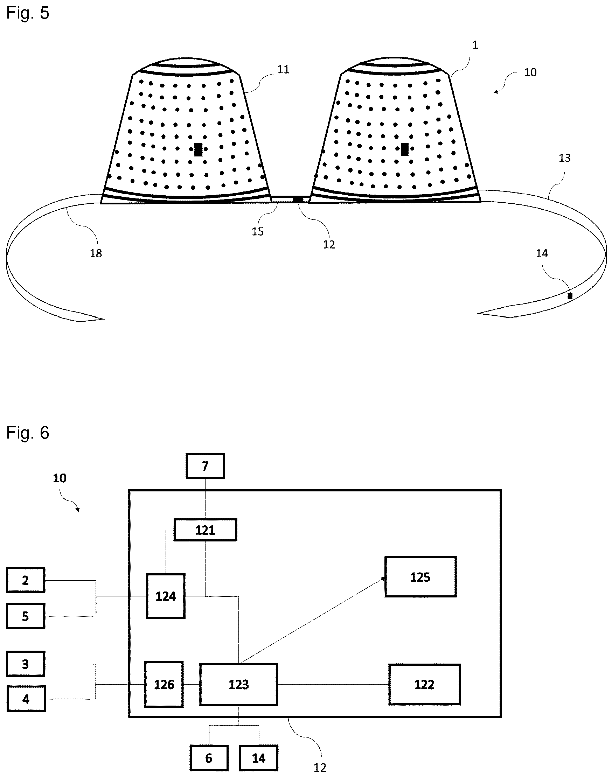

Les deux électrodes d'injection 2, 5 sont connectées à un générateur ou une source électrique 124, représentée sur la figure 6, de manière à injecter un courant alternatif entre les deux électrodes d'injection, à travers les tissus du sein. Préférentiellement, le courant est un courant alternatif. Alternativement, on peut imaginer l'utilisation d'un courant continu.

Les deux électrodes de mesures 3, 4 sont connectées à un mesureur de tension 126 pour mesurer l'impédance des tissus du sein entre ces deux électrodes de mesure 3, 4.

Dans un mode de réalisation, si une des électrodes d'injection 2, 5 est suffisamment large, son interface d'impédance peut être réduite de manière à rendre inutile l'électrode de mesure adjacente. Le mesureur de tension 126 peut alors être connecté à cette électrode d'injection pour permettre de simplifier le montage. Le bonnet 1 comprend alors une première électrode d'injection et une première électrode de mesure à une extrémité du bonnet (le sommet ou la base), ainsi qu'une troisième électrode à l'autre extrémité du bonnet, jouant le rôle d'électrode d'injection et d'électrode de mesure. De la même manière, le bonnet 1 peut comprendre une première électrode jouant le rôle d'électrode d'injection et d'électrode de mesure à une extrémité du bonnet (le sommet ou la base), ainsi qu'une deuxième électrode à l'autre extrémité du bonnet, jouant le rôle d'électrode d'injection et d'électrode de mesure.

Ainsi, la première électrode d'injection et la première électrode de mesure peuvent être la même électrode. De la même manière, la deuxième électrode d'injection et la deuxième électrode de mesure peuvent être la même électrode. Le jeu d'électrodes comprend ainsi au moins une première électrode au sommet d'au moins un bonnet et au moins une deuxième électrode au niveau de la base dudit au moins un bonnet. Le bonnet 1 peut comprendre deux électrodes 2, 5 uniquement.

Le dispositif médical 10 comprend au moins un capteur de température 6 disposé sur une branche 31, 32 de l'armature 30 du bonnet 1 pour mesurer la température cutanée de l'utilisatrice. En effet, la thermographie cutanée est un indicateur de détection des cancers. Ce paramètre varie aussi selon le cycle hormonal. Une zone d'un premier sein, plus chaude que la même zone dans le deuxième sein, pourra montrer une vasodilatation des vaisseaux sanguins et plus particulièrement une inflammation autour de la tumeur.

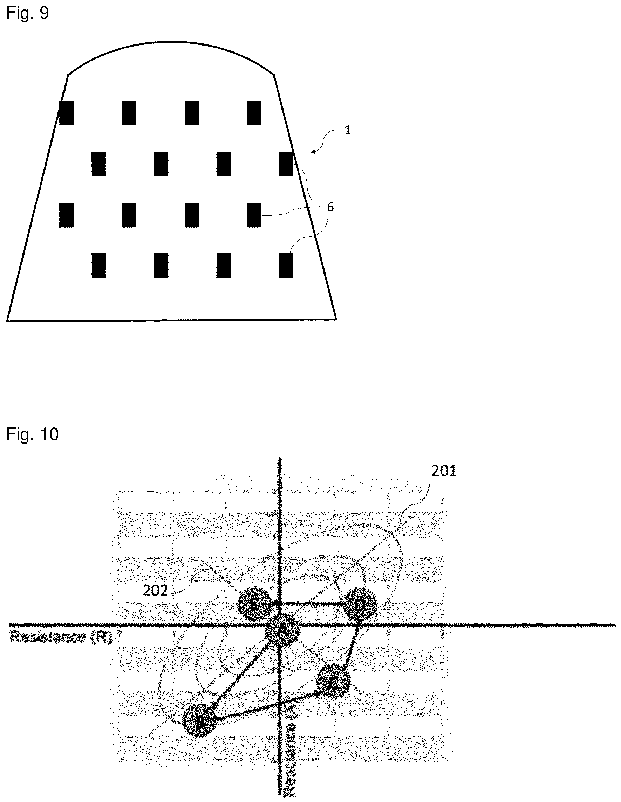

Le capteur de température 6 est agencé pour mesurer la température du sein de l'utilisatrice, comme représenté sur la figure 2. Comme illustré sur la figure 9, le bonnet 1 peut comprendre une pluralité de capteurs de température 6 disposés régulièrement sur la surface du bonnet pour mesurer la température du sein au plus d'endroit possible.

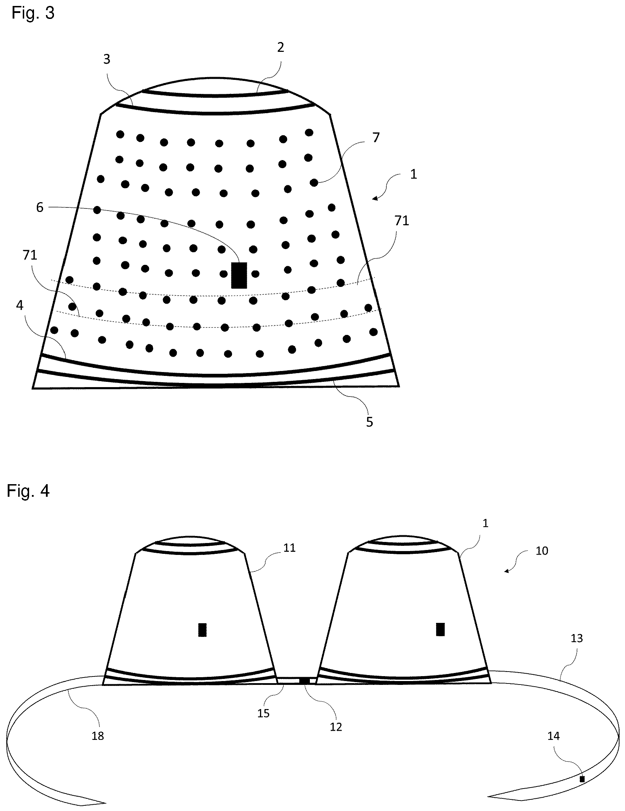

Les deux bonnets 1 , 11 du soutien-gorge 18 compris dans le dispositif médical 10 comprennent préférentiellement le même nombre de capteurs de température 6, comme représenté sur les figures 4 et 5. Préférentiellement, les capteurs de température 6 sont agencés sensiblement de la même manière d'un bonnet à l'autre de façon à ce que chaque capteur de température 6 puisse avoir un équivalent sur l'autre bonnet. Cet agencement permet avantageusement de comparer les températures d'un sein à l'autre et au même emplacement, pour détecter potentiellement une variation sur l'un des seins.

Le dispositif médical 10 comprend en outre au moins une branche latérale 13 comprenant un capteur de température corporelle 14 pour mesurer la température corporelle de l'utilisatrice.

Par « température corporelle », on entend la température cutanée de toute partie du corps exceptée celle de la poitrine de l'utilisatrice.

Ces capteurs de température corporelle 14 permettent de mesurer les variations de la température corporelle de l'utilisatrice, induites notamment par le cycle menstruel, afin de mesurer le bruit de la variation des températures mesurées sur le sein.

Préférentiellement, le dispositif comprend un, très préférentiellement au moins quatre capteurs de température corporelle 14. Préférentiellement, ces capteurs de température corporelle 14 sont disposés pour mesurer la température cutanée de l'utilisatrice au niveau des aisselles par exemple en étant agencés sur la branche 13 du soutien-gorge 18, comme représenté par les figures 4 et 5.

Dans un mode de réalisation particulier illustré sur les figures 3 et 5, le bonnet 1 peut comprendre un deuxième jeu d'électrodes. Le deuxième jeu d'électrodes permet de réaliser une détection spatiale ou en trois dimensions de la présence d'une tumeur. Selon le mode de réalisation, le deuxième jeu d'électrodes comprend une pluralité d'électrodes ponctuelles 7.

Les électrodes ponctuelles 7 sont préférentiellement de forme ronde, carrée ou rectangulaire. Les électrodes ponctuelles 7 comprennent une surface de contact inférieure à 7 cm² ou 2cm², préférentiellement inférieure à 1 cm² ou à 0,5 cm².

Les électrodes ponctuelles 7 peuvent comprendre des électrodes sèches telles que des électrodes textiles, silicones, des électrodes métalliques ou polymériques. Les électrodes ponctuelles 7 peuvent également comprendre des électrodes hydrogel Ag/AgCl.

Le deuxième jeu d'électrodes peut comprendre entre 10 et 200 électrodes ponctuelles, préférentiellement au moins vingt électrodes ponctuelles.

Les électrodes ponctuelles 7 sont agencées sur le bonnet 1 de manière à former un réseau ou un maillage d'électrodes.

Préférentiellement, les électrodes ponctuelles 7 sont espacées les unes des autres d'une distance comprise entre 5 et 25 mm, très préférentiellement entre 10 et 15 mm.

Les électrodes ponctuelles et/ ou les électrodes de mesure et d'injection peuvent être collées par une colle conductrice au textile du bonnet, préférentiellement sur sa surface intérieure c'est-à-dire la surface destinée à être en contact avec le sein. Les électrodes peuvent également être cousues au textile du bonnet, préférentiellement sur sa surface intérieure. En variante, les électrodes peuvent être intégrées ou liées par toute autre solution au textile d'un bonnet.

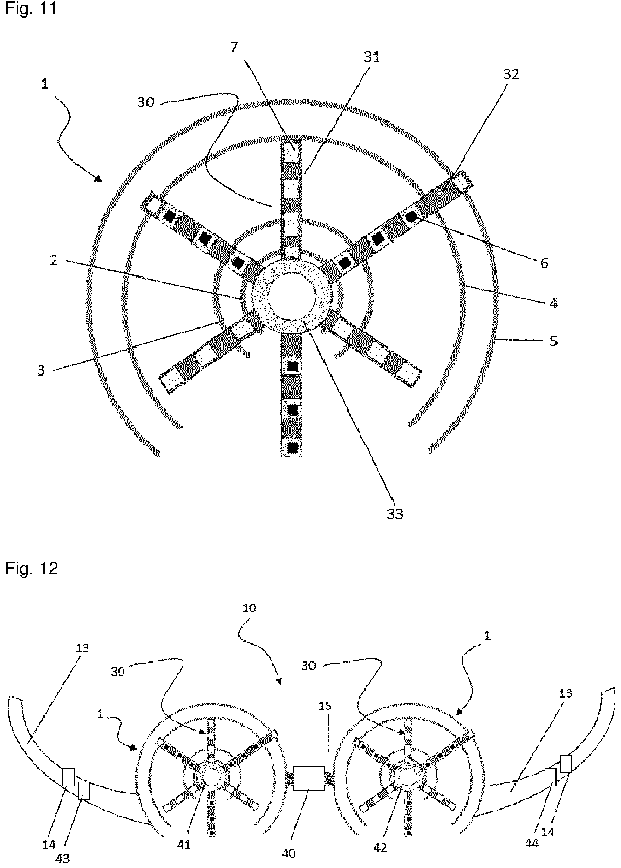

Le dispositif médical 10 comprend une armature 30 flexible comprenant au moins une branche 31, 32 s'étendant du sommet 16 à la base 17 du bonnet 1. Les électrodes 2, 3, 4, 5 sont fixées à au moins une branche 31, 32 de l'armature 30. Comme mentionné précédemment, le bonnet 1 du dispositif médical 10 ne comprend pas obligatoirement une enveloppe textile d'un soutien-gorge traditionnel. Les figures 11 et 12 illustrent ainsi un tel mode de réalisation.

Dans ce mode de réalisation, le bonnet 1 est formé par l'ensemble formé des électrodes et capteurs agencés sur une armature semi-élastique 30.

Selon ce mode de réalisation, l'armature 30 comprend un anneau rigide 33 central, d'où s'étendent six branches 31 , 32 semi-élastiques, formant une armature en forme d'étoile. La semi-élasticité des branches peut provenir du matériau choisi pour former les branches. En variante, toute autre solution est possible, comme le positionnement d'une ou plusieurs portions en matériau élastique entre d'autres portions plus rigides. Une portion peut par exemple présenter une forme en accordéon, lui permettant de s'étirer plus ou moins, de manière élastique. Avantageusement, ces branches remplissent une fonction de connexion électrique des électrodes et se présentent en un matériau conducteur ou comprennent des pistes conductrices, plus généralement comprennent des moyens conducteurs. Le matériau pour réaliser l'armature 30 peut être le matériau conventionnel d'un circuit imprimé (en anglais PCB pour « Printed Circuit Board »). En variante encore, la partie centrale de l'armature pourrait présenter une autre forme qu'un anneau rigide 33. De plus, l'armature pourrait comprendre tout autre nombre de branches, au moins une branche, de préférence au moins deux ou trois branches. Ces branches pourraient aussi présenter d'autres formes.

Le bonnet 1 comprend de plus des électrodes 2, 3, 4, 5, qui se présentent sous une forme circulaire ou annulaire ouverte. Les ouvertures de plusieurs électrodes peuvent être alignées entre elles, ou décalées autour du sein. En variante, cette forme annulaire d'une électrode peut être fermée, soit par le rapprochement jusqu'au contact des deux extrémités libres de l'électrode, par exemple dans le cas d'un sein de périphérie de dimension minimale, soit en variante par une électrode complètement annulaire, continue, sans extrémités libres. Ces électrodes se présentent ainsi dans des plans sensiblement parallèles. Chaque électrode s'étend avantageusement sur au moins 40%, voire sur au moins 60%, 70%, 80% ou 90%, de la périphérie du bonnet. Ces électrodes sont fixées à l'armature 30, plus précisément à une ou plusieurs branches 31 , 32 de cette armature. Cette fixation peut se faire par tout moyen, mécanique ou collage ou soudage. Deux électrodes 2, 3 de petit diamètre sont positionnées à proximité de l'anneau rigide 33, et deux électrodes 4, 5 de plus grand diamètre sont positionnées vers les extrémités des banches 31 , 32. En variante simplifiée, comme mentionné précédemment, le bonnet 1 pourrait ne comprendre que deux électrodes 2, 5.

Le bonnet 1 est ainsi destiné à un positionnement sur un sein, dans lequel l'anneau rigide 33 vient entourer le mamelon et les électrodes 4, 5 de plus grand diamètre se positionnent à la base du sein. Les branches 31 , 32 restent alors au contact du sein, s'étendant depuis le sommet jusqu'à la base. La flexibilité (élasticité ou semi-élasticité) des branches 31 et 32 leur permet d'épouser au mieux la forme du sein, et de garantir le positionnement de toutes les électrodes en contact avec le sein, et une proximité avec le sommet et la base du sein. Cet ensemble permet de plus de garantir un maintien autonome d'un bonnet sur un sein. Les électrodes ponctuelles fixées aux branches 31 et les capteurs de température fixés aux branches 32 se positionnent ainsi automatiquement au contact du sein.

Selon le mode de réalisation, en complément, trois branches 31 de l'armature 30 comprennent des électrodes ponctuelles 7 (trois à cinq électrodes ponctuelles 7 selon cette réalisation) pour des mesures complémentaires d'impédance, tel qu'expliqué précédemment en relation avec la figure 3. De plus, les trois autres branches 32 comprennent des capteurs de température 6 (trois à cinq capteurs de température 6 selon cette réalisation).

Enfin, selon le mode de réalisation, tout ou partie des moyens électroniques, représentés sur la figure 6, pourrait être positionné au centre de l'armature 30, par une fixation sur l'anneau rigide 33. Ce dernier pourrait par exemple héberger un microprocesseur et un multiplexeur.

En variante encore, les branches des armatures pourraient comprendre tout autre nombre de capteurs et électrodes. De plus, l'armature pourrait comprendre un autre nombre de branches.

Par exemple, dans un mode de réalisation plus complexe, l'armature 30 pourrait être constituée de 24 branches portant au total soixante électrodes ponctuelles 7 de bioimpédance et soixante capteurs de température 6, répartis comme suit :
- douze branches 31 portant chacune cinq électrodes ponctuelles 7 de bioimpédance ; et
- douze branches 32 portent chacune cinq capteurs de température 6.

Dans tous les cas, les branches 31 portant des électrodes ponctuelles 7 de bioimpédance peuvent être disposées en alternance avec les branches 32 portant des capteurs de température 6. En variante, toute autre disposition de ces branches est possible. De plus, une même branche pourrait comprendre au moins une électrode ponctuelle 7 et des capteurs de température 6.

En remarque, les branches 31 , 32 de l'armature comprennent les moyens conducteurs permettant de réaliser la liaison électrique entre les électrodes 2, 3, 4, 5, 7 et les éventuels capteurs 6, et les moyens électroniques du dispositif médical 10.

Les structures de l'armature 30 du bonnet décrit ci-dessus, en référence avec la figure 11 , peuvent être déclinées en plusieurs dimensions, afin de s'adapter aux différentes morphologies et tailles de seins pour garantir le meilleur positionnement de l'armature. Les dimensions paramétrables peuvent inclure :
- le diamètre intérieur de l'anneau rigide 33, et/ou
- la longueur des branches de l'armature 31 et 32, et/ou
- la longueur des électrodes circulaires 2, 3, 4, 5.

Le paramétrage dimensionnel de l'armature peut être établi en fonction de la profondeur des bonnets, classée de A à F, selon une classification internationale. A titre d'exemple, la longueur des électrodes circulaires 4, 5 peut être déclinée selon la profondeur des bonnets (de A à F). Autrement dit, six électrodes (prévues à la base du bonnet) de dimensions différentes permettraient de s'adapter au mieux à toutes les dimensions de seins. En variante simplifiée, une seule même électrode de dimension moyenne pourrait sensiblement convenir à toutes les dimensions de seins existants. Une telle électrode (prévue à la base du bonnet) pourrait à titre d'exemple présenter une longueur de 534 mm plus ou moins 20%. Naturellement, toutes les combinaisons intermédiaires sont possibles, et une solution pourrait reposer sur deux, trois, quatre ou cinq électrodes de dimensions différentes pour fabriquer des dispositifs médicaux selon l'invention adaptés à toutes les dimensions de seins.

Le dispositif médical 10 comprend en outre un multiplexeur ou un aiguilleur électrique 121 . L'aiguilleur électrique 121 permet d'aiguiller les tensions et les courants afin d'utiliser chaque électrode ponctuelle comme électrode d'injection, réceptrice ou comme masse.

Comme évoqué précédemment, le dispositif médical 10 comprend des moyens électroniques 12 pour gérer le fonctionnement du dispositif. Dans un mode de réalisation, les moyens électroniques 12 sont agencés sur le godet 15, c'est-à-dire la liaison entre les deux bonnets du soutien-gorge du dispositif, ou sur tout élément, par exemple à base d'une plaque de circuit imprimé, agencé entre les deux bonnets du dispositif médical 10. En variante, ces moyens électroniques peuvent être positionnés sur des bretelles, ou en zone arrière, par exemple dans le cas d'un dispositif médical associé à un soutien-gorge traditionnel. En variante encore, ils peuvent être positionnés sur toute zone plate, par exemple au-dessus des bonnets.

Les moyens électroniques 12 comprennent un multiplexeur 121 connecté à chaque électrode ponctuelle.

Le dispositif médical 10 comprend en outre une source électrique 124, de courant ou de tension. Cette source électrique 124 peut comprendre une batterie rechargeable. La source électrique 124 est connectée aux deux électrodes d'injection 2, 5 du premier jeu d'électrodes et optionnellement aux électrodes ponctuelles 7 du deuxième jeu d'électrodes.

Le dispositif médical 10 comprend également au moins un calculateur 123. Le calculateur 123 est connecté aux différents capteurs de températures 6, 14. Le calculateur 123 peut également recevoir les données de tensions mesurées par les électrodes de mesure 3, 4 et/ou par les électrodes ponctuelles 7. Le calculateur est connecté au mesureur de tension 126 et au multiplexeur 121 . Le calculateur peut également commander l'injection d'un courant entre les deux électrodes d'injection 2, 5 ou entre deux électrodes ponctuelles 7.

Dans un mode de réalisation, le bonnet 1 comprend des pistes conductrices textiles pour relier les différentes électrodes au générateur 124 et/ou au mesureur de tension 126. En variante, tout câble conducteur peut être agencé dans le dispositif médical 10. Selon le mode de réalisation de la figure 11 , cette connexion est assurée en partie par les branches 31 , 32 de l'armature 30.

Les moyens électroniques 12 peuvent de plus comprendre un transmetteur 125 capable de transmettre des informations à un ordinateur ou sur un réseau. Le transmetteur 125 est connecté au calculateur.

Dans un mode de réalisation non représenté, le dispositif médical comprend en outre un moyen de mesure de la concentration d'un composé dans le sang. Préférentiellement, ce moyen de mesure permet la mesure du taux d'oxygène dans le sang du sein. Le moyen de mesure peut comprendre une source d'un faisceau de lumière sur au moins une longueur d'onde et au moins un récepteur photodiode.

Préférentiellement, ladite longueur d'onde est comprise dans une plage allant de 500 nm et 1000 nm. T rès préférentiellement, la longueur d'onde est comprise dans une des plages de longueur d'onde centrées sur les longueurs d'onde adaptées pour mesurer au moins un des composés suivants : l'hémoglobine totale, la désoxyhémoglobine, l'oxyhémoglobine et la myoglobine.

La source d'un faisceau de lumière est agencée de manière à ce que le faisceau lumineux émis rencontre une partie du corps humain, préférentiellement le sein de l'utilisatrice, constituant une source de rétrodiffusion. Le récepteur photodiode est agencé de manière à recevoir ledit faisceau lumineux rétrodiffusé.

Pour mesurer une concentration d'un composé présent dans le sang, le dispositif met en oeuvre les étapes suivantes :
- émission d'au moins un faisceau lumineux à au moins une longueur d'onde ;
- la mesure de l'intensité de la lumière rétrodiffusée par le corps, préférentiellement par le sein, en fonction du temps ;
- le calcul de la concentration dudit composé présent à partir de l'intensité mesurée du au moins un faisceau lumineux rétrodiffusé.

Le dispositif médical 10 comprend un support 122 d'enregistrement de données, soit une mémoire électronique, lisibles par un ordinateur, sur lequel est enregistré un programme d'ordinateur pour le fonctionnement du dispositif médical 10. Le support 122 est un support d'enregistrement des données non-transitoire. Le support 122 est connecté au calculateur 123. Le calculateur 123 est ainsi capable de conduire les différents éléments matériels de manière à mettre en oeuvre les instructions enregistrées sur le support 122 d'enregistrement de données. Un programme d'ordinateur est enregistré sur ledit support d'enregistrement de données.

Ledit programme d'ordinateur ou les instructions enregistrées sur le support d'enregistrement de données permettent l'exécution d'une méthode d'estimation de la densité mammaire et/ou d'une méthode de calcul d'un score de risque de présence d'une tumeur cancéreuse telle que décrite ci-après.

Le support d'enregistrement de données 122 permet également de stocker les mesures et/ou les scores calculés, préférentiellement au moins jusqu'à ce que ces données soient transmises par le transmetteur 125.

La figure 12 illustre un dispositif médical 10 selon un mode de réalisation, comprenant deux bonnets 1 tels que décrit en référence à la figure 11 . Ces bonnets 1 sont reliés par un godet 15. De plus, deux extensions latérales, sous forme de deux branches latérales 13 semblables aux branches 31 , 32 de l'armature 30 des bonnets, s'étendent respectivement de part et d'autre des extrémités latérales, destinées à venir se positionner à proximité des aisselles d'une utilisatrice. Ces branches latérales 13 comprennent chacune un capteur de température 14, destiné à mesurer la température du corps en dehors d'un sein, pour former une valeur de référence de température.

De plus, dans ce mode de réalisation, les moyens électroniques sont répartis en différentes zones du dispositif, selon une architecture distribuée. Une telle approche peut avantageusement être implémentée dans tous les modes de réalisation. Elle présente l'avantage d'éviter l'utilisation d'un module électronique relativement volumineux comprenant de manière centralisée la totalité des moyens électroniques 12 du dispositif médical 10. Naturellement, en variante, une telle approche centralisée reste possible.

Notamment, dans le mode de réalisation, la gestion du dispositif médical (incluant le traitement des données et le pilotage des capteurs et électrodes) est répartie entre plusieurs microcontrôleurs 40, 41 , 42, 43, 44 intégrant au moins chacun un calculateur 123 et un support d'enregistrement 122. Ces microcontrôleurs 40, 41 , 42, 43, 44 sont respectivement positionnés sur le godet 15, sur chaque bonnet 1 , par exemple au niveau de la partie rigide centrale de l'armature 30, et au niveau de chaque branche latérale 13.

Le microcontrôleur central 40 positionné sur le godet 15 a pour rôle d'orchestrer le déroulement des mesures en coordonnant les microcontrôleurs locaux 41 , 42, 43, 44 et en recueillant les mesures effectuées par les microcontrôleurs locaux. Le microcontrôleur central 40 gère en outre l'interface utilisateur du système, notamment la communication sans fil avec un ordinateur distant, ou tout objet portable, sur lequel peut se trouver une interface homme machine pour permettre à l'utilisatrice de consulter les données provenant du dispositif médical et/ou paramétrer ce dispositif médical.

Chaque microcontrôleur local 41 , 42, 43, 44 est avantageusement placé au plus près des capteurs qu'il pilote. Il peut en être de même pour les multiplexeurs. Une telle disposition permet d'améliorer la qualité des mesures analogiques en minimisant l'atténuation du signal ainsi que le bruit.

Les microcontrôleurs 43, 44 disposés au niveau des aisselles d'une utilisatrice mettent en oeuvre les mesures de température corporelle. Ils gèrent chacun un unique capteur de température 14. Les microcontrôleurs 43,44 ainsi que le capteur de température 14 sont disposés sur les branches 13 du soutien-gorge, en regard des aisselles.

Les calculateurs 41 , 42 disposés au niveau des seins de l'utilisatrice mettent en oeuvre les mesures de bioimpédance et de température sur toute la surface du sein. Ils intègrent chacun un multiplexeur 121 , une source électrique 124 et tensiomètre 126, qui leur permettent de gérer chacun une pluralité d'électrodes (circulaires et ponctuelles) de bioimpédance. Ils sont disposés sur l'anneau rigide 33 de l'armature 30.

L'invention concerne un support d'enregistrement lisible par ordinateur comprenant des instructions qui, lorsqu'elles sont exécutées par un ordinateur, conduisent celui-ci à mettre en oeuvre une méthode d'estimation de la densité mammaire et/ou une méthode de calcul d'un score de risque de présence d'une tumeur cancéreuse. Ces méthodes sont naturellement mises en oeuvre sur la base d'un dispositif médical 10 tel que décrit précédemment.

On décrit ci-après une méthode d'estimation de la densité mammaire.

Une fois le bonnet 1 placé contre le sein de l'utilisatrice, un courant alternatif est appliqué entre les deux électrodes d'injection 2, 5. Un courant électrique est alors appliqué sur un segment entre ces deux électrodes, entre le sommet et la base du sein. De préférence, ce courant présente une fréquence comprise entre 4 et 500 kHz, voire entre 5 et 250 kHz. En complément, le courant est compris entre 8 et 64 µA, voire entre 8 et 32 µA.

La tension entre les deux électrodes de mesure 3, 4 est mesurée par le mesureur de tension 126. L'impédance et/ou la résistance des tissus du sein entre les deux électrodes de mesure 3, 4 est alors obtenue à partir de la loi d'Ohm.

À partir de cette impédance et/ou de cette résistance, une estimation de la densité mammaire est effectuée. Pour cela, on considère le sein comme un segment de corps ayant une masse de tissus adipeux et une masse de tissus mous (tissus fibro-glandulaires et conjonctifs). La valeur de l'impédance et/ou de la résistance permet d'obtenir la valeur de la masse de tissus adipeux par rapport à la masse de tissus mous. L'estimation peut comprendre le classement de la densité mammaire entre un certain nombre de catégories correspondant au pourcentage de tissus mous. On peut, par exemple, utiliser la classification de BI-RADS comprenant 4 classes décrites ci-après.

La classe 1 : masse de tissus mous inférieure à 25%. La classe 2 : masse de tissus mous comprise entre 25% et 50%. La classe 3 : masse de tissus mous comprise entre 51 et 75%. La classe 4 : masse de tissus mous supérieure à 75%. On peut imaginer une autre classification, par exemple en 6 classes. Le dispositif médical permet dans tous les cas de procéder à une estimation précise du pourcentage de densité mammaire, et non uniquement une estimation grossière selon la classification BI-RADS.

La variation de cette densité durant le cycle menstruel et avec l'âge peut permettre d'établir le bruit et l'augmentation du risque de l'avènement d'une tumeur.

Cette estimation peut ainsi être comparée à une valeur de référence. La valeur de référence peut être une estimation de la densité mammaire réalisée précédemment, par exemple au même moment du cycle hormonal de l'utilisatrice, sur le même sein et/ou sur l'autre sein. De manière alternative, la valeur de référence peut comprendre une courbe étalon développée à partir de mesures précédentes sur une population saine selon l'âge et/ou selon le moment du cycle menstruel.

Dans un mode de réalisation, un premier score de risque de présence d'une tumeur cancéreuse est alors calculé en fonction de l'estimation de la densité mammaire et/ou en fonction de la comparaison entre la densité mammaire estimée et la valeur de référence.

Un deuxième score peut être calculé pour mesurer le risque de présence de tumeur, directement à partir de mesures d'impédance du premier jeu d'électrodes.

Les mesures d'impédance comprennent la détermination d'une résistance (partie réelle de l'impédance) et d'une réactance (partie imaginaire de l'impédance).

Les mesures de résistance et de réactance peuvent être comparées à des valeurs de référence. On peut également comparer une valeur calculée à partir desdites mesures de résistance et/ou de réactance.

Ces valeurs peuvent être des valeurs relatives, par exemple des valeurs relatives par rapport à des valeurs de référence.

Les valeurs de référence peuvent être obtenues à partir de valeurs réalisées sur une population de sujets sains ou à partir de mesures précédentes sur l'utilisatrice, par exemple à un même moment du cycle menstruel.

Le deuxième score est alors fonction de la valeur relative de la réactance et/ou de la valeur relative de la résistance. Ce deuxième score permet de quantifier le risque de présence d'une tumeur dans le sein. Ce deuxième score ne permet pas de localiser la tumeur puisque les mesures sont effectuées dans tout le volume du sein, depuis la base jusqu'au sommet, plus exactement depuis la première électrode de mesure jusqu'à la deuxième électrode de mesure.

Dans un premier exemple, il a été réalisé des essais de mesure d'impédance à l'aide d'un dispositif selon un mode de réalisation de l'invention illustré sur la figure 1 sur des fantômes.

Les fantômes 1 à 3 consistent en un mélange d'un premier matériau représentatif des tissus mous présents dans le sein et d'un deuxième matériau représentatif du tissu adipeux présent dans le sein.

Le fantôme 1 comprend 100% du premier matériau représentatif des tissus mous (par rapport à la somme du premier et deuxième matériau). Le fantôme 2 comprend 50% du premier matériau. Le fantôme 3 comprend 20% du premier matériau.

Plusieurs mesures d'impédance (Zr) ont été réalisés sur chacun de ces fantômes. Les résultats obtenus (moyenne des résultats et écart-type en unité arbitraire) sont reportés dans les colonnes 2 à 4 du tableau 1 ci-dessous.

**[Tableau 1]**

| | Fantôme 1 (100%) | Fantôme 2 (50%) | Fantôme 3 (20%) | Fantômes avec tumeur |
|---|---|---|---|---|
| Moyenne Zr | 24,9 | 27,9 | 37,2 | 19,3 |
| Ecart-type Zr | 2,9 | 0,3 | 0,0 | 1,7 |

Il peut être observé, que les valeurs d'impédance diminuent avec l'augmentation de la masse de tissus mous dans le sein.

Il peut donc bien être réalisé une estimation précise de la densité mammaire suite à des mesures d'impédance à l'aide du dispositif selon un mode de réalisation de la présente invention.

Il a été réalisé plusieurs fantômes comprenant une sphère d'un matériau représentatif d'une tumeur cancéreuse. Les tissus cancéreux ayant une conductivité supérieure (et donc une résistance inférieure) à celle des tissus sein, les fantômes comprenant une telle sphère ont été réalisés avec une base de 100% de matériau représentatif des tissus mous.

Chaque fantôme avait une sphère de différent diamètre, allant de T à 8'.

Les valeurs obtenues lors de mesure d'impédance sont reportées sur la 5^{ème} colonne du tableau 1.

Il peut être remarqué que les valeurs obtenues sont encore inférieures aux valeurs obtenues pour le fantôme 1.

Il peut donc bien être réalisé une détection d'un risque de présence d'une tumeur cancéreuse suite à des mesures d'impédance à l'aide du dispositif selon un mode de réalisation de la présente invention.

Dans un deuxième exemple, on utilise une méthode BIVA (pour l'anglicisme « Bioimpedance Vector Analisis »).

Dans un schéma illustré sur la figure 10, on représente les scores de résistance en abscisse et les scores de réactance en ordonnée. Les scores de réactances peuvent être obtenus par l'écart entre une valeur mesurée et la moyenne divisé par l'écart type, la moyenne et l'écart type étant calculés à partir de mesures précédentes.

La méthode BIVA définit deux axes, un premier axe 201 représentatif du taux hydrique des tissus du sein et un deuxième axe 202 représentatif du taux de masse de cellules actives ou le taux de masse de tissus mous selon le type de schéma BIVA, dans le volume du sein.

En fonction de la zone dans laquelle se trouve la mesure effectuée, on peut en déduire une pathologie du sein.

Par exemple, la mesure A représente un sein sain.

Le secteur B (valeurs relatives de la réactance et de la résistance significativement négatives) représente un sein contenant une surcharge hydrique, pouvant être due à un sepsis (un syndrome d'infection générale et grave de l'organisme par des agents pathogène), une surcharge de fluide due à une variation durant le cycle menstruel ou à une inflammation pouvant être notamment due à la présence d'une tumeur. Le secteur B peut aussi représenter des tissus dont les cellules présentent une diminution de leur fonction réactive, soit une augmentation de leurs caractéristiques capacitives, comme c'est le cas des cellules des tumeurs cancéreuses.

Le secteur C (valeurs relatives de la réactance et de la résistance significativement respectivement négatives et positives) représente un sujet dont le sein comprend un taux important de tissus adipeux.

Le secteur D (valeurs relatives de la réactance et de la résistance significativement positives) représente un sein ayant une répartition normale de tissus mous par rapport aux tissus adipeux avec une activité membranaire significative pouvant être due à une phase spécifique du cycle hormonal.

Enfin, le secteur E (valeurs relatives de la réactance et de la résistance significativement respectivement positives et négatives) représente un sein avec un fort taux de tissus mous fibro-glandulaires et conjonctifs.

Selon le secteur Biva vers lequel se dirige le vecteur, on estime s'il y a une évolution des caractéristiques capacitives des membranes cellulaires et résistives des tissus, indiquant une potentielle tumeur cancéreuse.

De plus, selon le secteur Biva vers lequel se dirige le vecteur, on sait s'il y a augmentation de la densité mammaire ou non.

Un troisième score de risque de présence d'une tumeur cancéreuse peut être calculé à partir de mesures de température cutanée du sein de l'utilisatrice.

Ces mesures de température sont effectuées par le premier capteur 6 de température agencé sur le bonnet 1 du dispositif médical 10. Préférentiellement, le dispositif médical 10 comprend une pluralité de capteurs 6 de température sur le bonnet 1 , comme illustré sur la figure 9. La mesure de la température cutanée du sein de l'utilisatrice comprend alors une mesure de température pour chaque capteur 6 de température du bonnet.

Dans un mode de réalisation, la mesure comprend une mesure de température pour chaque capteur 6 de température des deux bonnets.

Cette mesure de température est ensuite comparée à une ou plusieurs températures de référence. La température de référence peut comprendre ou être déterminée en fonction d'une ou plusieurs mesures réalisées précédemment. La température de référence peut comprendre une moyenne des mesures réalisées précédemment par le même capteur.

La température de référence peut aussi comprendre la température mesurée sur le deuxième bonnet par un capteur de température équivalent, préférentiellement au même moment ou dans un intervalle de temps inférieur à une certaine durée, de préférence sans qu'un élément ne vienne modifier la température pendant l'intervalle (par exemple une douche ou une augmentation du rythme cardiaque), par exemple d'une heure ou moins. Deux capteurs équivalents sont deux capteurs agencés chacun sur un bonnet différent, mais chacun au même endroit par rapport à un sein. La température mesurée est donc ainsi comparée à la température mesurée sur l'autre sein au même endroit. Un écart significatif entre les deux seins peut être un indice de la présence d'une tumeur.

Dans un mode de réalisation, cette variation de température du sein ou d'une région du sein est comparée à la variation de température corporelle de l'utilisatrice.

On peut mesurer la température du corps de l'utilisateur, notamment grâce aux capteurs de température corporelle 14 prévus pour mesurer la température corporelle de l'utilisatrice à un endroit en dehors des seins.

Cette mesure de la température corporelle peut être comparée à ou aux mesures de température cutanée du sein. L'étude de la variation de la température corporelle permet de réduire le bruit lié aux variations de la température cutanée du sein, pouvant être causé par une activité sportive, le cycle menstruel ou le moment de la journée, par exemple après une douche.

En fonction de la comparaison de la température mesurée à la valeur de référence, un troisième score de risque est calculé. Ce troisième score est calculé en fonction de la comparaison de la température du sein par rapport à la valeur de référence. Ce troisième score de risque est corrélé à la variation de la température d'une zone du sein. Ce score peut aussi être calculé en fonction de la variation de la température corporelle. Par exemple, on peut soustraire à la variation de la température mesurée en un point la variation de la température corporelle. Ce troisième score permet de détecter un risque de tumeur. En effet, l'inflammation causée par les tumeurs cancéreuses produit de la chaleur et peut être détectée.

Dans un mode de réalisation décrit ci-dessous, le premier, deuxième et/ou troisième score peuvent être déterminés de manière locale dans le volume du sein.

Dans ce mode de réalisation, le dispositif médical 10 comprend un réseau ou un maillage d'électrodes ponctuelles 7, le dispositif médical 10 permet de réaliser une cartographie du volume du sein par bioimpédance. De préférence, le dispositif médical 10 comprenant un réseau ou un maillage d'électrodes comprend également une pluralité de capteurs de température agencés pour mesurer la température du sein. Un tel bonnet est illustré sur la figure 9 sur laquelle les électrodes ne sont pas représentées dans un souci de clarté. Préférentiellement la pluralité de capteurs de température forme un réseau ou un maillage de capteurs de température.

Ce réseau ou maillage d'électrodes ponctuelles et /ou de capteurs de température autorise avantageusement la détermination d'un score de risque similaire au premier, au deuxième et/ou au troisième score de risque de façon spatiale. Ce mode de réalisation permet ainsi la détermination de la densité mammaire, de la température et/ou de la détection et/ou de la spécification de tumeur cancéreuse de manière spatiale.

Cette cartographie peut être réalisée de deux manières décrites ci-après dans un premier et un deuxième mode d'exécution.

Dans un premier mode d'exécution, la cartographie est réalisée par une technique de tomographie par impédance électrique (appelée « EIT » en anglais).

Pour effectuer ces mesures, le deuxième jeu d'électrodes comprend des électrodes ponctuelles 7 disposées en plusieurs cercles 71 à différents intervalles comme illustré sur la figure 3.

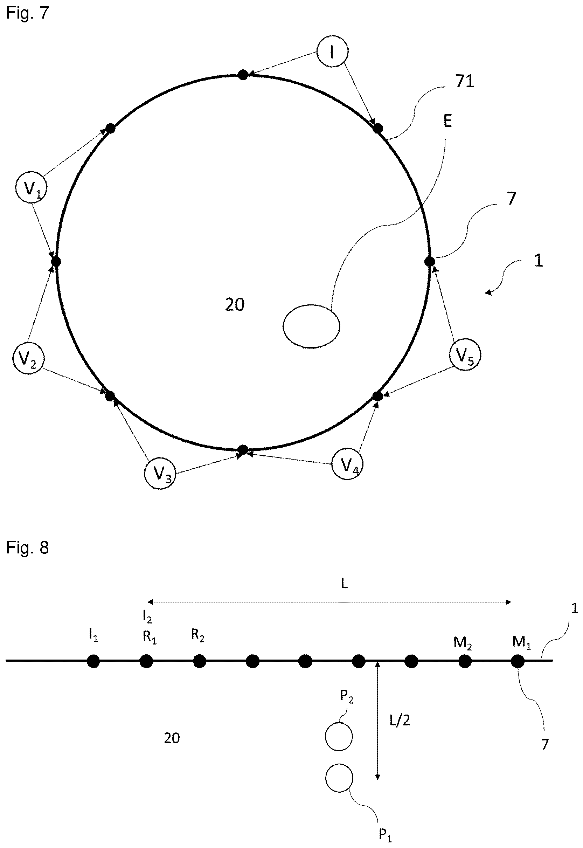

Une vue en coupe montrant un cercle 71 d'électrodes ponctuelles 7 comprenant huit électrodes ponctuelles autour des tissus du sein 20 est illustré sur la figure 7. On applique un courant alternatif entre une première paire I d'électrodes adjacentes et on mesure les potentiels de surface V-i , V2, V3, V4, V5, au niveau des cinq autres paires d'électrodes adjacentes. L'application de la loi d'Ohm permet dès lors de déterminer l'impédance bioélectrique entre la première paire I et les autres paires d'électrodes à partir du courant appliqué connu et des tensions mesurées. Ensuite, la paire d'électrodes adjacente est utilisée pour l'application de courant suivante et les cinq autres mesures de tension sont effectuées. L'emplacement des paires d'électrodes entre lesquelles on applique un courant et les paires d'électrodes de mesure se déplacent ainsi successivement. Un tour complet permet d'obtenir des profils de tension sur les huit positions d'électrodes, composées chacune de cinq valeurs de mesures de tension. Les quarante valeurs ainsi obtenues forment une trame et permettent de reconstituer une image transversale de tomographie par impédance électrique.

Une modification régionale de l'impédance E de l'utilisatrice (ici, une tumeur cancéreuse) entraîne une modification de chacun des huit profils de tension constituant la trame. Quel que soit le point d'application du courant, l'augmentation régionale E de l'impédance entraîne une augmentation des tension « derrière » la région dont l'impédance augmente (V4 dans le cas illustré sur la figure 7).

En compilant les profils de tension, et en superposant les huit profils obtenus, on obtient une image représentant la région E dont l'impédance augmente.

Plus le nombre d'électrodes ponctuelles 7 disposées sur le cercle est important, plus la précision est accrue.

On peut ainsi répéter la méthode avec plusieurs cercles 71 d'électrodes ponctuelles 7 pour obtenir plusieurs images transversales et ainsi, obtenir une cartographie d'impédance du volume du sein 20.

Dans un deuxième mode d'exécution, la cartographie est réalisée par mesure d'impédance tripolaire. La méthode d'impédance tripolaire est illustrée sur la figure 8.

A l'aide d'un aiguilleur de tensions 121 , on injecte un courant à travers les tissus du sein 20 entre les électrodes ponctuelles h et Mi . Mi est alors connectée à la masse. Avec une troisième électrode Ri , de préférence, l'électrode adjacente à li entre Mi et l-i , on effectue une mesure d'impédance entre Ri et la masse Mi du courant injecté.

La profondeur Pi supposée de la mesure est de la moitié de la distance L entre Ri et Mi , c'est-à-dire L/2.

On peut ainsi réaliser plusieurs mesures en rapprochant ou en éloignant les électrodes sélectionnées pour obtenir une valeur à différents endroits et à différentes profondeurs. Par exemple, on peut brancher la masse sur l'électrode M2, injecter un courant entre les électrodes I2 et M2, et réaliser une mesure entre R2 et M2.

Le centre du segment R2M2 est le même que celui du segment R1M1 mais la distance est plus faible, ce qui permet d'obtenir une mesure d'impédance à une profondeur P2 plus faible que L/2.

Par une méthode d'analyse de l'impédance bioélectrique, par exemple par une méthode d'analyse vectorielle, il est possible de détecter une variation tissulaire, et donc, une variation de densité locale ou un risque local de présence d'une tumeur.

En répétant les mesures et en faisant varier les électrodes et les distances, on obtient une cartographie d'impédance.

Dans un mode de réalisation, on peut réaliser des scores de risque de présence d'une tumeur en mode local, permettant de localiser une potentielle tumeur cancéreuse et d'être plus précis dans la probabilité du risque. Le score de risque locale peut être similaire au premier et au deuxième calculs explicités précédemment.

Le calculateur 123 détermine un score de risque combiné de présence d'une tumeur dans le sein en combinant le score de risque déterminé à partir des mesures d'impédance et/ou de résistance et le score de risque déterminé à partir des mesures de température.

Le score de risque combiné correspond à un quatrième score de risque, déterminé par le calculateur 123 en combinant le score de risque déterminé à partir des mesures d'impédance et/ou de résistance et le score de risque déterminé à partir des mesures de température.

Si le premier, le deuxième, le troisième et/ou le quatrième score de risque atteint une valeur-seuil, le dispositif médical 10 transmet, par le transmetteur 125, une information d'alerte à un ordinateur distant.

Dans un mode de réalisation, le premier, le deuxième, le troisième et/ou le quatrième score est fonction de la concentration d'un composé dans le sang mesuré par le moyen de mesure d'un composé présent dans le sang décrit ci-avant.

L'ordinateur distant peut comprendre un téléphone de type smartphone de l'utilisatrice. L'invention peut permettre d'envoyer à un professionnel de santé une alerte sur une hausse du score de risque de l'utilisatrice, et optionnellement, les valeurs des mesures de densité mammaire, de température et/ou d'impédance.

Préférentiellement les différentes mesures d'impédance et/ou de température peuvent être effectuées au moment de la pose du soutien-gorge.

Le dispositif selon l'invention permet avantageusement de monitorer régulièrement une utilisatrice, et d'augmenter les chances de détection précoce d'une formation de tumeur cancéreuse dans le sein de l'utilisatrice, ainsi que d'en avertir immédiatement un professionnel de santé pour réaliser des examens au plus vite.

## Revendications

1. Dispositif médical (10) comprenant un ou deux bonnets (1) comprenant chacun une ou deux électrodes (2, 3) agencées au sommet (16) du bonnet (1), une ou deux électrodes (4, 5) agencées à la base (17) du bonnet (1), ces électrodes étant aptes à des mesures d'impédance et/ou de résistance entre le sommet du sein et la base du sein;
**caractérisé en ce que** le dispositif comprend une armature (30) flexible comprenant au moins une branche (31, 32) s'étendant du sommet a la base du bonnet (1), les électrodes (2, 3, 4, 5) étant fixées à au moins une branche (31, 32) de l'armature (30);
et **en ce que** le dispositif comprend en outre au moins un capteur de température (6) disposé sur une branche (31, 32) de l'armature (30) du bonnet (1) ;
et au moins une branche (13) latérale comprenant un capteur de température (14) apte à un positionnement à proximité de l'aisselle d'une utilisatrice,
et **en ce que** le dispositif comprend un calculateur (123) configuré pour :
acquérir des mesures d'impédance et/ou de résistance entre le sommet du sein et la base du sein au moyen des électrodes :
acquérir des mesures de température au moyen dudit au moins un capteur de température disposé sur une branche de l'armature et dudit capteur de température de la branche latérale ;
déterminer un score de risque de présence d'une tumeur dans le sein à partir des mesures d'impédance et/ou de résistance ;
déterminer un score de risque de présence d'une tumeur dans le sein à partir des mesures de température ; et
déterminer un score de risque combiné de présence d'une tumeur dans le sein en combinant lesdits scores de risque.

2. Dispositif médical (10) selon la revendication précédente, **caractérisé en ce que** le jeu d'électrodes comprend une première électrode de mesure (3) et une première électrode d'injection (2) disposées à proximité du sommet du bonnet (1) et/ou une deuxième électrode d'injection (5) et une deuxième électrode de mesure (4) à proximité de la base du bonnet (1).

3. Dispositif médical (10) selon l'une des revendications précédentes, **caractérisé en ce que** chaque électrode, particulièrement au moins une électrode au niveau de la base du bonnet (1), s'étend au moins partiellement autour du bonnet (1), préférentiellement s'étend sur au moins 40%, voire sur 20 au moins 60%, 70%, 80%, ou 90% de la périphérie du bonnet.

4. Dispositif médical (10) selon l'une des revendications précédentes, **caractérisé en ce que** le bonnet (1) comprend au moins une électrode à proximité de la base du bonnet (1) de forme totalement ou partiellement annulaire, et au moins une électrode à proximité du sommet du bonnet (1), et l'armature flexible permet le maintien desdites électrodes au contact d'un sein et le maintien du bonnet (1) au contact d'un sein.

5. Dispositif médical (10) selon l'une des revendications précédentes, **caractérisé en ce qu'**il comprend une source électrique (124) pour appliquer une tension entre une première électrode et une deuxième électrode et comprend un élément de mesure de tension (126) pour mesurer la tension entre la première et la deuxième électrode.

6. Dispositif médical (10) selon la revendication 3, **caractérisé en ce que** le bonnet (1) comprend deux électrodes de mesure (3, 4) agencées entre deux électrodes d'injection (2, 5).

7. Dispositif médical (10) selon l'une des revendications précédentes, **caractérisé en ce que** le bonnet (1) comprend en outre une pluralité d'électrodes ponctuelles (7), préférentiellement agencées en réseau, pour réaliser une cartographie d'impédance dans le volume du sein.

8. Dispositif médical (10) selon l'une des revendications précédentes, **caractérisé en ce que** l'armature (30) comprend une partie centrale rigide d'où s'étendent plusieurs branches (31, 32) semi-élastiques, lesdites branches s'étendant depuis le sommet du bonnet (1) en direction de la base du bonnet (1), formant une armature (30) en forme d'étoile, sur laquelle sont fixées les électrodes.

9. Dispositif médical (10) selon l'une des revendications précédentes, **caractérisé en ce qu'**il comprend tout ou partie des caractéristiques complémentaires suivantes :
- Au moins une électrode ponctuelle (7) de bioimpédance disposée sur une branche (31, 32) de l'armature (30) du bonnet (1) ;
- Au moins une électrode à proximité de la base du bonnet (1) et/ou à proximité du sommet du bonnet (1) de forme totalement ou partiellement annulaire, de longueur telle qu'elle s'étend sur au moins 40%, voire sur au moins 60%, 70%, 80% ou 90%, de la périphérie du bonnet ;
- Au moins un microcontrôleur (41, 42) dispose sur le bonnet, notamment sur une partie rigide centrale de l'armature (30) ;
- Des moyens conducteurs d'au moins une branche (31, 32) de l'armature (30) du bonnet pour connecter électriquement les électrodes du bonnet (1) vers un module de gestion.

10. Dispositif médical (10) selon l'une des revendications précédentes, **caractérisé en ce qu'**il comprend deux bonnets (1), et des moyens électroniques (12) agencés sur un godet (15) et sur chaque bonnet (1), lesdites électrodes étant relies électriquement à tout ou partie desdits moyens électroniques par l'intermédiaire de moyens conducteurs d'au moins une branche (31, 32) de l'armature (30) de chaque bonnet (1).

11. Dispositif médical (10) selon l'une des revendications précédentes, **caractérisé en ce qu'**il comprend des éléments matériels (2, 3, 4, 5) et/ou logiciels configurés pour mettre en œuvre les étapes suivantes :
- une mesure d'une impédance et/ou d'une résistance entre le sommet du sein et la base du sein par l'intermédiaire dudit jeu d'électrodes ;
- une estimation de la densité mammaire à partir de cette mesure par un moyen logiciel :
- une comparaison de ladite estimation a une valeur de référence par un moyen logiciel :
- un calcul d'un score de risque en fonction de cette comparaison par un moyen logiciel.

## Patentansprüche

1. Medizinische Vorrichtung (10), umfassend ein oder zwei Körbchen (1), die jeweils eine oder zwei Elektroden (2, 3), die an der Spitze (16) des Körbchens (1) angeordnet sind, und eine oder zwei Elektroden (4, 5), die an der Basis (17) des Körbchens (1) angeordnet sind, umfassen, wobei diese Elektroden für Impedanz- und/oder Widerstandsmessungen zwischen der Brustspitze und der Brustbasis geeignet sind;
**dadurch gekennzeichnet, dass** die Vorrichtung ein flexibles Gestell (30) umfasst, das mindestens einen Ast (31, 32) umfasst, der sich von der Spitze zur Basis des Körbchens (1) erstreckt, wobei die Elektroden (2, 3, 4, 5) an mindestens einem Ast (31, 32) des Gestells (30) befestigt sind;
und dass die Vorrichtung ferner mindestens einen Temperatursensor (6) umfasst, der an einem Ast (31, 32) des Gestells (30) des Körbchens (1) angeordnet ist;
und mindestens einen seitlichen Ast (13), der einen Temperatursensor (14) umfasst, der zur Positionierung in der Nähe der Achselhöhle einer Trägerin geeignet ist,
und dass die Vorrichtung eine Recheneinheit (123) umfasst, die dazu konfiguriert ist:
mittels der Elektroden Impedanz- und/oder Widerstandsmessungen zwischen der Brustspitze und der Brustbasis zu erfassen;
mittels des mindestens einen an einem Ast des Gestells angeordneten Temperatursensors und des Temperatursensors des seitlichen Astes Temperaturmessungen zu erfassen;
aus den Impedanz- und/oder Widerstandsmessungen einen Risikoscore für das Vorliegen eines Tumors in der Brust zu bestimmen;
aus den Temperaturmessungen einen Risikoscore für das Vorliegen eines Tumors in der Brust zu bestimmen; und
durch Kombination der genannten Risikoscores einen kombinierten Risikoscore für das Vorliegen eines Tumors in der Brust zu bestimmen.

2. Medizinische Vorrichtung (10) nach dem vorhergehenden Anspruch, **dadurch gekennzeichnet, dass** der Elektrodensatz eine erste Messelektrode (3) und eine erste Injektionselektrode (2), die in der Nähe der Spitze des Körbchens (1) angeordnet sind, und/oder eine zweite Injektionselektrode (5) und eine zweite Messelektrode (4) in der Nähe der Basis des Körbchens (1) umfasst.

3. Medizinische Vorrichtung (10) nach einem der vorhergehenden Ansprüche, **dadurch gekennzeichnet, dass** sich jede Elektrode, insbesondere mindestens eine Elektrode im Bereich der Basis des Körbchens (1), zumindest teilweise um das Körbchen (1) herum erstreckt, vorzugsweise über mindestens 40 %, oder sogar über mindestens 60 %, 70 %, 80 % oder 90 % des Umfangs des Körbchens.

4. Medizinische Vorrichtung (10) nach einem der vorhergehenden Ansprüche, **dadurch gekennzeichnet, dass** das Körbchen (1) mindestens eine Elektrode in der Nähe der Basis des Körbchens (1) mit vollständig oder teilweise ringförmiger Form und mindestens eine Elektrode in der Nähe der Spitze des Körbchens (1) umfasst, und das flexible Gestell es ermöglicht, dass die genannten Elektroden in Kontakt mit einer Brust gehalten werden und das Körbchen (1) in Kontakt mit einer Brust gehalten wird.

5. Medizinische Vorrichtung (10) nach einem der vorhergehenden Ansprüche, **dadurch gekennzeichnet, dass** sie eine elektrische Quelle (124) zum Anlegen einer Spannung zwischen einer ersten Elektrode und einer zweiten Elektrode und ein Spannungsmesselement (126) zum Messen der Spannung zwischen der ersten und der zweiten Elektrode umfasst.

6. Medizinische Vorrichtung (10) nach Anspruch 3, **dadurch gekennzeichnet, dass** das Körbchen (1) zwei Messelektroden (3, 4) umfasst, die zwischen zwei Injektionselektroden (2, 5) angeordnet sind.

7. Medizinische Vorrichtung (10) nach einem der vorhergehenden Ansprüche, **dadurch gekennzeichnet, dass** das Körbchen (1) ferner eine Vielzahl von Punktelektroden (7) umfasst, die vorzugsweise in einem Array angeordnet sind, um eine Impedanzkartierung im Volumen der Brust zu erstellen.

8. Medizinische Vorrichtung (10) nach einem der vorhergehenden Ansprüche, **dadurch gekennzeichnet, dass** das Gestell (30) einen starren zentralen Teil umfasst, von dem sich mehrere halbelastische Äste (31, 32) erstrecken, wobei sich die genannten Äste von der Spitze des Körbchens (1) in Richtung der Basis des Körbchens (1) erstrecken und ein sternförmiges Gestell (30) bilden, an dem die Elektroden befestigt sind.

9. Medizinische Vorrichtung (10) nach einem der vorhergehenden Ansprüche, **dadurch gekennzeichnet, dass** sie alle oder einige der folgenden zusätzlichen Merkmale umfasst:
- Mindestens eine Bioimpedanz-Punktelektrode (7), die an einem Ast (31, 32) des Gestells (30) des Körbchens (1) angeordnet ist;
- Mindestens eine Elektrode in der Nähe der Basis des Körbchens (1) und/oder in der Nähe der Spitze des Körbchens (1) mit vollständig oder teilweise ringförmiger Form, deren Länge derart ist, dass sie sich über mindestens 40 %, oder sogar über mindestens 60 %, 70 %, 80 % oder 90 % des Umfangs des Körbchens erstreckt;
- Mindestens einen Mikrocontroller (41, 42), der am Körbchen, insbesondere an einem starren zentralen Teil des Gestells (30), angeordnet ist;
- Leitende Mittel mindestens eines Astes (31, 32) des Gestells (30) des Körbchens zur elektrischen Verbindung der Elektroden des Körbchens (1) mit einem Verwaltungsmodul.

10. Medizinische Vorrichtung (10) nach einem der vorhergehenden Ansprüche, **dadurch gekennzeichnet, dass** sie zwei Körbchen (1) und elektronische Mittel (12) umfasst, die an einem Mittelsteg (15) und an jedem Körbchen (1) angeordnet sind, wobei die genannten Elektroden mittels leitender Mittel mindestens eines Astes (31, 32) des Gestells (30) jedes Körbchens (1) elektrisch mit allen oder einigen der genannten elektronischen Mittel verbunden sind.

11. Medizinische Vorrichtung (10) nach einem der vorhergehenden Ansprüche, **dadurch gekennzeichnet, dass** sie Hardwareelemente (2, 3, 4, 5) und/oder Softwareelemente umfasst, die dazu konfiguriert sind, die folgenden Schritte durchzuführen:
- eine Messung einer Impedanz und/oder eines Widerstands zwischen der Brustspitze und der Brustbasis mittels des genannten Elektrodensatzes;
- eine Schätzung der Brustdichte anhand dieser Messung durch ein Softwaremittel;
- einen Vergleich der genannten Schätzung mit einem Referenzwert durch ein Softwaremittel;
- eine Berechnung eines Risikoscores in Abhängigkeit von diesem Vergleich durch ein Softwaremittel.

## Claims

1. A medical device (10) comprising one or two cups (1) each comprising one or two electrodes (2, 3) arranged at the apex (16) of the cup (1), and one or two electrodes (4, 5) arranged at the base (17) of the cup (1), these electrodes being suitable for impedance and/or resistance measurements between the apex of the breast and the base of the breast;
**characterized in that** the device comprises a flexible frame (30) comprising at least one branch (31, 32) extending from the apex to the base of the cup (1), the electrodes (2, 3, 4, 5) being fixed to at least one branch (31, 32) of the frame (30);
and **in that** the device further comprises at least one temperature sensor (6) arranged on a branch (31, 32) of the frame (30) of the cup (1);
and at least one lateral branch (13) comprising a temperature sensor (14) suitable for being positioned close to the armpit of a user,
and **in that** the device comprises a computing unit (123) configured to:
acquire impedance and/or resistance measurements between the apex of the breast and the base of the breast by means of the electrodes;
acquire temperature measurements by means of said at least one temperature sensor arranged on a branch of the frame and of said temperature sensor of the lateral branch;
determine a risk score for the presence of a tumor in the breast from the impedance and/or resistance measurements;
determine a risk score for the presence of a tumor in the breast from the temperature measurements; and
determine a combined risk score for the presence of a tumor in the breast by combining said risk scores.

2. The medical device (10) according to the preceding claim, **characterized in that** the set of electrodes comprises a first measurement electrode (3) and a first injection electrode (2) arranged close to the apex of the cup (1) and/or a second injection electrode (5) and a second measurement electrode (4) close to the base of the cup (1).

3. The medical device (10) according to one of the preceding claims, **characterized in that** each electrode, particularly at least one electrode at the base of the cup (1), extends at least partially around the cup (1), preferably extends over at least 40%, or even at least 60%, 70%, 80%, or 90% of the periphery of the cup.

4. The medical device (10) according to one of the preceding claims, **characterized in that** the cup (1) comprises at least one electrode of totally or partially annular shape close to the base of the cup (1), and at least one electrode close to the apex of the cup (1), and the flexible frame allows said electrodes to be kept in contact with a breast and the cup (1) to be kept in contact with a breast.

5. The medical device (10) according to one of the preceding claims, **characterized in that** it comprises an electrical source (124) for applying a voltage between a first electrode and a second electrode and comprises a voltage measurement element (126) for measuring the voltage between the first and the second electrode.

6. The medical device (10) according to claim 3, **characterized in that** the cup (1) comprises two measurement electrodes (3, 4) arranged between two injection electrodes (2, 5).

7. The medical device (10) according to one of the preceding claims, **characterized in that** the cup (1) further comprises a plurality of point electrodes (7), preferably arranged in an array, for producing an impedance mapping within the volume of the breast.

8. The medical device (10) according to one of the preceding claims, **characterized in that** the frame (30) comprises a rigid central part from which several semi-elastic branches (31, 32) extend, said branches extending from the apex of the cup (1) towards the base of the cup (1), forming a star-shaped frame (30) to which the electrodes are fixed.

9. The medical device (10) according to one of the preceding claims, **characterized in that** it comprises all or some of the following additional features:
- At least one bioimpedance point electrode (7) arranged on a branch (31, 32) of the frame (30) of the cup (1);
- At least one electrode close to the base of the cup (1) and/or close to the apex of the cup (1), of totally or partially annular shape, of a length such that it extends over at least 40%, or even at least 60%, 70%, 80% or 90%, of the periphery of the cup;
- At least one microcontroller (41, 42) arranged on the cup, in particular on a central rigid part of the frame (30);
- Conductive means of at least one branch (31, 32) of the frame (30) of the cup for electrically connecting the electrodes of the cup (1) to a management module.

10. The medical device (10) according to one of the preceding claims, **characterized in that** it comprises two cups (1), and electronic means (12) arranged on a gore (15) and on each cup (1), said electrodes being electrically connected to all or some of said electronic means by means of conductive means of at least one branch (31, 32) of the frame (30) of each cup (1).

11. The medical device (10) according to one of the preceding claims, **characterized in that** it comprises hardware elements (2, 3, 4, 5) and/or software elements configured to implement the following steps:
- a measurement of an impedance and/or a resistance between the apex of the breast and the base of the breast by means of said set of electrodes;
- an estimation of breast density from this measurement by a software means;
- a comparison of said estimate with a reference value by a software means;
- a calculation of a risk score as a function of this comparison by a software means.
